(19) Europäisches Patentamt
European Patent Office
Office européen des brevets

(11) Publication number: **0 209 870**
**A2**

## (12) EUROPEAN PATENT APPLICATION

(21) Application number: 86109942.2

(22) Date of filing: 19.07.86

(51) Int. Cl.⁴: **A 61 K 7/035**
**A 61 K 7/46, C 08 J 9/28**

(30) Priority: 22.07.85 US 757251

(43) Date of publication of application:
28.01.87 Bulletin 87/5

(84) Designated Contracting States:
BE DE FR GB IT NL SE

(71) Applicant: DeSOTO, INC.
1700 South Mt. Prospect Road
Des Plaines Illinois 60018(US)

(72) Inventor: Brown, Wallace H.
1303 Gilbert
Downers Grove, Ill.60515(US)

(72) Inventor: Vandeberg, John T.
415 West Oakwood Drive
Barrington, Ill. 60010(US)

(72) Inventor: Poklacki, Erwin S.
1223 S. Fernandez
Arlington Heights, Ill. 60005(US)

(72) Inventor: Dachniwskyj, Maryam L.
1320 Cambia Drive 7208
Schaumburg, Ill.60193(US)

(74) Representative: Reitzner, Bruno, Dr. et al,
Patentanwälte Dipl.-Ing. R. Splanemann Dr. B. Reitzner
Tal 13
D-8000 München 2(DE)

(54) Cosmetic composition with sustained release of vaporizable chemical.

(57) Vesiculated beads of cross-linked resin containing an average of at least 2 thin-walled foraminous cells to provide communication between the vesicles within the beads and the exterior of said beads and to allow liquid to move into and out of the beads, have a vaporizable chemical incorporated therein. The vaporizable chemical loaded into the beads after they are formed and is preferably a liquid perfume. The beads containing the perfume or other vaporizable chemical can be incorporated into dry powder, cream or lotion compositions.

-1-

# COSMETIC COMPOSITION WITH SUSTAINED RELEASE OF VAPORIZABLE CHEMICAL

## DESCRIPTION

### Technical Field

This invention relates to cosmetic compositions characterized by the sustained release of a vaporizable chemical, normally a liquid. While various vaporizable products are contemplated, such as insect repellents, deodorants, and the like, the invention particularly contemplates perfumes.

### Background Art

Cosmetic compositions containing vaporizable chemicals are in common use, the most common vaporizable products being perfumes. One difficulty with these compositions is the unsatisfactorily steep decline in the concentration of the desired vapor with time. Thus, in a perfume-containing composition, the fragrance initially applied may be too strong and offensive. On the other hand, some time later when it is important for the perfume to be fully effective, it may be too faint for such purpose. Similarly, an insect repellent when initially applied may be so vaporous as to chase away people as well as insects, but some hours later the vapor may be too weak to prevent an insect's bite.

Perfumes suffer from a further disadvantage as a result of the fact that they are usually blends of essences, each with a somewhat different vapor pressure. Individual essences rarely have an attractive odor, but the perfumer is skilled in blending a plurality of different essences to produce a desirable fragrance. Unfortunately, if the fragrance is attractive when initially applied, then it may be very ordinary an hour or two later when the more volatile components are in short supply. On the

-2-

other hand, if the perfumer balances the essences for peak aroma some time after application, the customer may not select that fragrance because the perfume is sampled as freshly applied.

It will now be evident that it is important to be able to apply the vaporizable chemical in a way which flattens the curve of concentration vs time and which makes more uniform the variation of composition with time. These difficulties have presented the art with a perplexing problem which has not previously been satisfactorily resolved.

One possibility is to encapsulate the perfume within some sort of a tiny capsule, but this has presented a very limited choice. While the perfume is in the capsule, it has little fragrance. After the capsule has been ruptured, the full contents of the capsule become available, and the same problems apply as previously discussed. It is possible to periodically disrupt some of the microcapsules to release the perfume as needed, but this requires time and attention, like reapplying a normal perfume during the party.

It is also possible to absorb the volatile agent onto a powder component, but in that instance much of the volatile agent is on the surface available for immediate evaporation, so the sustained release effect is a limited one. Also, selective adsorption by the powder particles can distort the normal evaporation of a multicomponent mixture.

Description of Invention

In accordance with this invention, the vaporizable chemical is incorporated into preformed vesiculated beads of cross-linked resin, the beads containing an average of at least 2, more usually at least 5, thin-walled foraminous cells which allow liquid to move into and out of the beads. These cells preferably include at least some cell walls which are discontinuous on electron microscope examination to provide communication between the vesicles within the bead and the exterior of the bead and thus enable movement of liquid by capillary action, though this action may not require openings large enough to be seen. The very thin walls of the cells (about 0.2 micron) are concluded to be foraminous, for water contained therein is easily removed by simple exposure to air even though no fissure large enough for the electron microscope to pick up is found in many of the cells.

While capillary action seems to be significant to the movement which takes place in this invention, the very thin walls of the cells may enable other actions to take place. Thus, if a solid vaporizable chemical is loaded into the beads as a liquid solution, and then the solvent is evaporated to leave solid chemical within the beads, this solid chemical can later leave the beads by sublimation. These solid vaporizable chemicals are illustrated herein by camphor and menthol which can be incorporated into powders which provide a slow and durable cooling effect as the solid slowly vaporizes. This is useful, for example, in a foot powder.

This invention thus includes a process of incorporating a vaporizable chemical into hollow plastic beads in a manner permitting this chemical to leave the beads when they are spread on a surface. More particularly, it is found that the vesiculated beads can be loaded with vaporizable chemical after bead formation is complete, and the loaded material largely remains within the beads when the beads are

-4-.

covered, as when they are stored in a closed container alone or together with other materials prior to use. When the loaded beads are later applied as a thin layer or dust (onto the skin or other surface), the vaporizable chemical is slowly released.

In this way, the volatile agent on the surface is initially present in only a small amount which limits the concentration of vapor in the air above the treated surface. As time progresses, the agent is constantly renewed from the beads as it evaporates from the treated surface so that the liquid remains available on that surface over an extended period of time. As a result, the initial concentration of vaporizable component in the air is reduced in comparison with normal application, and the concentration of vaporizable component in the air after an hour or more is greater than would be provided by normal application. The beads in this invention thus act as a reservoir of supply to prolong the presence of the vaporizable chemical and make its action more uniform with time.

The vaporizable chemical in liquid form can be loaded into the vesiculated beads by simply mixing the two together and holding them together in admixture with one another to allow the vaporizable chemical to enter the cells of the beads. Agitation can be used, but is not usually necessary. While all the liquid can be taken up, this is not necessary unless a powder product is desired.

The vaporizable chemical may be used in combination with inert volatile organic solvent, such as an alcohol like ethyl alcohol or propyl alcohol, or a ketone like acetone or methyl ethyl ketone. When a solvent is used, all or a portion of the solvent can be removed after the solution of the chemical has moved into the beads by allowing it to evaporate. In this way, the usual large initial concentration of active vapor pulled into the air by the evaporation of the solvents usually employed for perfume application is diminished. Of course, the solvent removed in the bead-loading process contains some perfume or other vaporizable chemical, and they can be condensed and reused.

When the vaporizable chemical is an easily flowable liquid, and many perfume oils and emollients are of this character, then the use of solvent is not required. On the other hand, when the vaporizable chemical is a viscous liquid or when it is a sublimable solid, then volatile solvent can be used to increase fluidity. Thus, menthol and camphor can be used in ethyl alcohol solution.

These vesiculated beads with the vaporizable component within them can be applied alone or in combination with other components in a liquid lotion or cream, or in a powder composition in combination with talc or other powdered component, such as a clay, for instance kaolin clay. The composition may include colorants or other additions for special purpose. Indeed, it is well known that perfumes are incorporated into all sorts of cosmetic compositions, and that perfume in this invention would be incorporated after it had been loaded into vesiculated beads, as has been described.

The terms "cream" and "lotion" are well known in the cosmetic art. A "cream" is an emulsion of oil droplets in an aqueous phase or water droplets in an oleaginous phase having a sem-solid consistency. A "lotion" has the same emulsion structure, but the emulsion has sufficient fluidity

-6-

to be pourable. Of course, the emulsion may be simple or complex, and high viscosity liquids possess intermediate properties, so there is some overlap between these terms. Particularly where the water phase is the continuous one, the addition of water to a cream composition can provide a lower viscosity lotion composition.

In the practice of this invention, the beads are associated with the oil phase of the emulsion. The vesiculated beads to be useful in this invention must be substantially unground, for significant grinding breaks up the beads and impairs their usefulness. The beads may vary in average size of from about 1 micron to about 30 microns, but beads having an average size of from 3 microns to 20 microns are preferred. These beads preferably average at least about 5 vesicles per bead.

The beads in this invention are preferably substantially free from water, and this denotes a water content of less than about 0.2% by weight. The dried beads can be provided by removing water from the water-wet paste of vesiculated beads which is provided when these beads are produced by polymerization in aqueous emulsion. Water removal can be by simple exposure to air at room temperature, by passing heated air, typically at a temperature of 100°F. to 120°F. over the beads, by tumbling the wet beads in a rotating structure such as a pipe or centrifuge basket while air is passed through the beads, by dropping them through a tower with drying gas moves upwardly therethrough, by vacuum drying, by using an azeotroping solvent, or in any other desired manner.

It should be noted that the beads are usually present in an aqueous cake which includes the surfactants and colloidal material involved in their production. These tend to cause agglomeration when simple drying is used, but this can be avoided by washing the beads with water one or more times to remove these adhesive agents with the wash water. Solvent washing is also permitted, and the water can be azeotropically removed by heating the solvent. Some of the solvent can be allowed to remain to help load the beads with the volatile liquid.

The water-wet paste which is treated in the above manner is formed when excess water is drained from the aqueous suspension obtained when the beads are prepared in aqueous suspension, a surfactant and/or protective colloid being needed to maintain the suspension while the beads are produced. A typical paste is called a cake, and it contains about 65% water while appearing to be dry.

The beads in this invention can be pigmented or unpigmented, depending upon the cosmetic application which is intended. When pigmentation is desired, the pigment can be incorporated into the walls of the beads as they are formed, and one can also associate with the preformed beads a pigment which is incompatible with the azeotropic organic solvent. Thus, one can add a water-based pigment, such as lamp black, to the aqueous paste either before or after the addition of organic azeotropic solvent to be present while the water is azeotropically removed. This causes the added pigment to deposit upon and adhere to the surface of the beads.

The vesiculated beads are produced in an aqueous suspension which is drained to form a water-wet paste. The procedure for producing these beads involves polymerization in aqueous emulsion in the presence of surfactants and, preferably also, protective colloids. Protective colloids such as polyvinyl alcohol and hydroxyethyl cellulose are frequently used.

The preferred vesiculated beads are styrene-cross-linked unsaturated polyester resins. These are made into a vesiculated bead in conventional fashion, as illustrated by U.S. Pat. No. 3,879,314. Various other patents are of interest to the formation of vesiculated beads useful in this invention, particular attention being directed to U.S. patents Nos. 3,822,224, 3,923,704 and 3,933,579. This last-named patent describes the vesiculated beads which are preferred herein, namely, those having a ratio of granular diameter to mean vesicle diameter of at least 5:1, a vesicle volume of from 5% to 95% of the volume of the granule, and not more than about 60% pigment, by volume.

The vesiculated beads used herein have a highly cross-linked polymeric body which is preferably constituted by a carboxyl-functional unsaturated polyester resin cross-linked with an ethylenically unsaturated monomer copolymerizable therewith. The unsaturation in the polyester is preferably maleate unsaturation, these polyesters being themselves well known and illustrated hereinafter. It is preferred that the polyester have an acid value of 10 to 45 mgm KOH per gm.

During the production process, water migrates into the polymeric beads to swell them, and the polymer in the bead walls polymerizes at the same time. As a result, it is normal to have some of the cell walls fracture, and the extent of fracturing can be controlled by controlling the polymerization process. The more polymerization can be delayed, the

-9-

greater the number of cell walls which are either not present or disrupted. In this way, the proportion of volatile liquid which can be held by the vesiculated beads can be varied.

The unsaturated monomers used for cross-linking are also well known and are water insoluble monomers typically illustrated by styrene or vinyl toluene. The polyesters and monomers which may be used are more fully discussed in U.S. Pat. No. 3,879,314 which shows the production of vesiculated beads using a water-soluble polyamine containing at least three amine groups per molecule and having a dissociation constant in water (pKa value) of 8.5-10.5, typically illustrated by diethylene triamine. The polyamine is used in a concentration providing 0.3 to 1.4 amine groups per polyester carboxyl group. It is preferred to have from 35% to 45% of the unsaturated polyester cross-linked with from 55% to 65% of styrene.

While the vesiculated beads used herein may be pigmented or unpigmented, this invention will be illustrated using pigmented beads. Suitable pigmented vesiculated beads in accordance with this invention are illustrated in U.S. Patent No. 3,879,314 issued April 22, 1975, see particularly Example II. By proceeding in accordance with said Example II and using a polyester of 18% phthalic anhydride, 37% maleic anhydride and 45% propylene glycol dissolved in styrene to form a solution containing 41.8% of the polyester, vesiculated beads pigmented with titanium dioxide, anatase, to contain about 43.2% pigment are provided. These beads have an average size of about 9 microns and contain an average of more than 10 cells per bead, and are the beads used in the Examples of this application.

-10-

The invention is illustrated in the examples which follow. All parts and proportions herein are by weight unless otherwise specified.

Example 1

An aqueous slurry of vesiculated beads has the bulk of its water content removed by decantation to provide an aqueous paste (termed a beadcake) containing 35 parts of vesicular beads, 64.5 parts water, 0.18 parts of a 75% solution of sodium dioctyl sulfosuccinate (the American Cyanamid surfactant, Aerosol OT may be used), 0.28 parts polyvinyl alcohol and 0.04 parts hydroxy ethyl cellulose. These surfactants and colloids are typical residues of bead production and they would cause agglomeration if the water were removed by simple drying. The vesiculated beads are composed of 56.0% resin and 44.0% titanium dioxide. The bead resin is an unsaturated polyester containing propylene glycol/maleic anhydride/phthalic anhydride in proportions of 3.72/2.06/4.22, and this polyester is dissolved in styrene to provide a 58/42 ratio of styrene to polymers. To form the beads, the mixture of polyester and styrene is dispersed in water with the aid of surfactants and protective colloids in the proportions noted and copolymerized in the presence of a quaternary ammonium salt to cause vesicles to form as described in U.S. Pat. No. 3,879,314. The resulting vesiculated beads are in water slurry, and this is drained to provide the beadcake starting material.

The beadcake is then dispersed in 50% of its volume of deionized water with agitation and allowed to settle, the water being decanted to lower the concentration of adhesive agents. This operation is repeated several times with about 300% by volume of deionized water to obtain water-containing beads

-11-

substantially free of agglomerating adhesive agents. These water-wet beads are dried by spreading them on a tray and passing warm air at a temperature of about 110°F. thereover. The water on and within the beads evaporates, and the drying process is effective, enabling one to obtain beads which are sufficiently dry to be combined with isocyanate-functional liquids without inducing reaction with water.

Example 2

To a stainless steel kettle equipped with an agitator, add 47.5 pounds of fragrance MF #0925 (which is a fragrance oil obtainable from V. Mane Fils of Fairfield, NJ) and then slowly add 47.5 pounds of the dried beads obtained in Example 1 and mix until uniform. Then add 5.0 pounds of fumed silica and mix to provide a dry free-flowing powder.

This powder can be dusted onto skin to provide a durable application of perfume, or it can be mixed with talc or clay or any powder cosmetic to provide a perfumed cosmetic preparation in dry powder form. It can also be mixed into cream or lotion cosmetic products to add its fragrance thereto.

Example 3

To a stainless steel kettle equipped with an agitator, add 71.25 pounds of isopropyl alcohol and then add 95 pounds of N,N-diethyl toluamide. To the solution so-provided, slowly add 285 pounds of the dried beads obtained in Example 1 and mix until uniform. Then add 48.75 pounds of fumed silica and mix to provide a dry free-flowing powder.

This powder can be dusted onto skin to provide a long lasting insect repellent action. It can also be incorporated into cream or lotion cosmetic products to add a durable insect repelling action to such cream or lotion.

CLAIMS

1. Vesiculated beads of cross-linked resin, said beads containing an average of at least 2 thin-walled foraminous cells to provide communication between the vesicles within said bead and the exterior of said beads to allow liquid to move into and out of said beads, said beads containing vaporizable chemical.

2. Vesiculated beads as recited in claim 1 in which at least some of said cell walls are discontinuous as determined by electron microscopy, and there are an average of at least 5 thin-walled cells per bead.

3. Vesiculated beads as recited in claim 1 in which said beads are constituted by a carboxyl-functional unsaturated polyester resin cross-linked with an ethylenically unsaturated monomer copolymerizable therewith and have a ratio of granular diameter to mean vesicle diameter of at least 5:1, a vesicle volume of from 5% to 95% of the volume of the granule, and not more than about 60% pigment, by volume.

4. Vesiculated beads as recited in claim 3 in which said polyester resin contains maleate unsaturation and is cross-linked with styrene or vinyl toluene.

5. Vesiculated beads as recited in claim 1 in which said vaporizable chemical comprises emollient oil.

6. Vesiculated beads as recited in claim 1 in which said vaporizable chemical comprises perfume.

7. A dry powder composition comprising talc or clay and the vesiculated beads recited in claim 1.

8. A cream or lotion containing the vesiculated beads recited in claim 1.

9.    A cream or lotion as recited in claim 8 in which said beads are associated with the oil phase of said cream or lotion.

10.    A process of incorporating a vaporizable chemical into hollow plastic beads in a manner permitting said vaporizable chemical to leave said beads when said beads are spread on a surface comprising, mixing substantially dry vesiculated beads of cross-linked resin containing an average of at least 2 thin-walled foraminous cells with said vaporizable chemical in liquid form, and holding said beads in admixture with said vaporizable chemical in liquid form to allow said vaporizable chemical to enter the cells of said beads.